# FASCICULE DE BREVET EUROPEEN

(11) **EP 0 565 496 B1**
(45) Date de publication et mention de la délivrance du brevet: **20.11.1996**
(21) Numéro de dépôt: 93830148.8
(22) Date de dépôt: 07.04.1993
(51) Int. Cl.: G01N 21/86, G01N 33/36

(54) **Procédé pour la détection de la variation de densité d'un tissu et dispositif pour sa mise en oeuvre**
Verfahren und Vorrichtung zur Erfassung von Dichtedifferenzen in einem Gewebestück
Method and apparatus for detecting density differences in a fabric

(30) Priorité: 10.04.1992 IT FI920088
(43) Date de publication de la demande: 13.10.1993
(73) Titulaire: SOLIS S.r.l., I-50029 Impruneta (Firenze) Fract. Tavarnuzze (IT)
(72) Inventeur: Migliorini, Pier Lorenzo, I-52028 Terranuova Bracciolini (Arezzo) (IT)
(74) Mandataire: Martini, Lazzaro

(56) Documents cités:
- DE-C- 3 325 127
- US-A- 3 859 537
- US-A- 4 192 242
- US-A- 4 249 081
- US-A- 4 376 400
- PATENT ABSTRACTS OF JAPAN vol. 16, no. 114 (C-921)23 Mars 1992 & JP-A-03 286 796 ( TAKATORI HAITETSUKU ) 17 Décembre 1991
- PATENT ABSTRACTS OF JAPAN vol. 15, no. 421 (C-878)25 Octobre 1991 & JP-A- 03 173 597 ( TAKATORI HAITETSUKU ) 26 Juillet 1991

## Description

La présente invention a pour objet un procédé pour la détection de la variation de densité d'un tissu et un dispositif pour sa mise en oeuvre.

Il est connu que, dans l'industrie textile, le problème de la détection de discontinuités sur des tissus, dues par exemple à la couture ou à la superposition de tissus de différentes caractéristiques ou encore aux variations de densité d'un même tissu, revêt une importance fondamentale.
En particulier, ce problème est particulièrement ressenti dans la production des collants et dans la finition des bas.

Il est connu, en effet, qu'une phase particulièrement importante du processus de fabrication d'un collant est celle concernant le positionnement automatique des bas avant d'exécuter leur coupe longitudinale; ce positionnement est rendu possible grâce à la détection de la ligne de jarretière de chacun des deux bas: la ligne de jarretière étant produite par la variation de densité du tissu en correspondance de la zone de jonction entre le corps et la jambe du bas.

Les procédés et les dispositifs connus pour la détection de la ligne de jarretière prévoient l'utilisation de moyens d'entraînement du bas sur des supports correspondants pour permettre de tendre temporairement le tissu, c'est-à-dire pour le rendre exempt de plis, et permettre à un capteur optique approprié de détecter ladite ligne.

Les désavantages qui dérivent de l'utilisation desdits procédés et dispositifs connus consistent essentiellement dans le fait que, étant donné qu'il est nécessaire de tendre le tissu sur les moyens de support et que ces derniers présentent une longueur inférieure à la distance entre la ligne de jarretière et le bord élastique du bas, il est obligatoire tout d'abord d'amener la ligne de jarretière sur les moyens de support et, après sa détection, de la ramener dans une position prédéterminée, en dehors et au-delà des moyens de support; que ledit déplacement nécessite un temps d'exécution considérable qui est imposé également par la sensibilité relative du capteur.

Le document US 4.247.081 décrit un dispositif pour la détection de défauts dans des éléments tubulaires, dans lequel chaque élément à contrôler tourne autour de son axe longitudinal et est déplacé dans la direction axiale respective au cours du contrôle, de manière à réaliser un mouvement relatif entre l'élément et un détecteur optique fixe. Le dispositif précité comprend, en combinaison: un capteur électronique pour détecter une portion d'élément ayant une longueur supérieure à celle correspondant à la translation axiale qui se produit simultanément à la rotation d'un tour de l'élément: ledit capteur étant habilité à émettre un signal électrique approprié simultanément à la détection d'un défaut; des moyens pour le comptage des signaux émis par ledit capteur; un temporisateur connecté auxdits moyens de comptage pour permettre de rapporter le nombre de défauts détectés dans l'unité de temps au temps d'exécution de chaque mesure; des moyens de contrôle, connectés audit temporisateur et auxdits moyens de comptage, pour l'émission d'un signal électrique correspondant si le nombre des défauts détectés dans l'unité de temps est supérieur à une valeur prédéterminée; des moyens d'identification des défauts, destinés à reconnaître la présence de signaux répétitifs dans un intervalle de temps prédéterminé. Le signal instantané émis par le capteur suite à la détection d'un défaut est amplifié, comparé à un signal de contrôle, filtré et enfin compté. Si le nombre de défauts, c'est-à-dire des signaux ainsi émis par le capteur, dans l'unité de temps est supérieur à une valeur prédéterminée, cette condition commande l'arrêt du dispositif et éventuellement de la machine qui produit l'élément contrôlé.
Mais ce dispositif connu n'est pas utilisable de manière avantageuse dans la fabrication des collants et dans le finissage des bas, principalement en considération du fait que le signal instantané, émis par le capteur et pris comme base de référence décisionnelle pour les moyens qui commandent l'arrêt de l'élément contrôlé et/ou le signalement de la condition de défaut détecté, n'est pas traité, c'est-à-dire que le calcul de sa moyenne n'est pas effectué, de manière à permettre la détection fiable de simples variations de densité dans le tissu d'un bas: le manque de fiabilité provient du fait que même un éventuel faux pli ou un pli similaire du tissu serait considéré de la même manière que n'importe quel défaut et ceci n'est admis ni dans la fabrication des collants, ni dans le finissage des bas.
Le but de la présente invention est d'éliminer les inconvénients mentionnés.

Ce résultat a été atteint, conformément à l'invention, en adoptant un procédé de traitement qui comporte: - le déplacement du tissu à traiter par rapport à un capteur optique à réflexion fixe, destiné à détecter la variation de densité du tissu;
- le prélèvement du signal de sortie dudit capteur et la transmission, en parallèle, du signal ainsi prélevé à un circuit de tampon à amplificateurs opérationnels et à des moyens pour calculer la moyenne et retarder ledit signal;
- la transmission des signaux de sortie, respectivement du circuit tampon et desdits moyens pour calculer la moyenne et retarder le signal prélevé sur les moyens optiques, à l'entrée d'un amplificateur différentiel;
- la transmission du signal de sortie dudit amplificateur différentiel à l'entrée d'un comparateur du signal par rapport à un signal connu et prédéterminé;
- la transmission du signal de sortie dudit comparateur aux moyens opérationnels pour le traitement successif du tissu lorsque le signal en sortie dudit amplificateur est d'intensité supérieure à une valeur prédéterminée.

Et pour mettre en oeuvre ledit procédé, on utilise un dispositif avec un capteur optique pour la détection de la ligne de discontinuité du tissu, lequel est caractérisé en ce qu'il comprend:
- un circuit de tampon à amplificateurs opérationnels avec l'entrée connectée de manière électrique à la sortie du capteur optique;
- des moyens pour calculer la moyenne et retarder le signal prélevé à la sortie du capteur optique;
- des moyens d'amplification différentielle des signaux de sortie du circuit tampon et des moyens pour calculer la moyenne et retarder le signal prélevé sur la sortie du capteur optique;
- des moyens pour comparer le signal provenant des moyens d'amplification différentielle avec un signal connu et prédéterminé;
- des moyens pour utiliser le signal de sortie des moyens de comparaison.

Les avantages obtenus grâce à la présente invention consistent essentiellement en ce qu'il est possible de détecter la discontinuité, c'est-à-dire une ligne de démarcation de la densité d'un tissu, sans soumettre ce dernier à un étirement particulier, ce qui permet d'obtenir une simplification considérable de construction et de fonctionnement et une fiabilité extrêmement élevée; qu'il est possible de détecter des variations de densité même très faibles; que le temps nécessaire pour obtenir la détection d'une discontinuité sur le tissu s'avère fortement diminué grâce à l'absence des phases de fonctionnement relatives au tendage du tissu avant qu'il ne soit disposé en correspondance du capteur de détection.

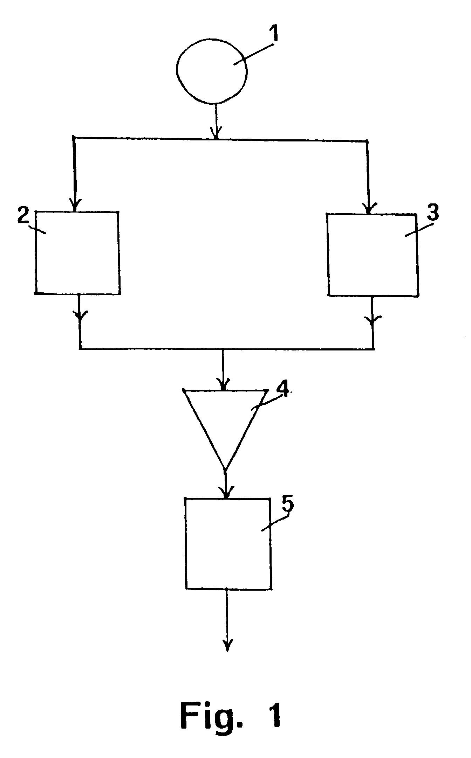

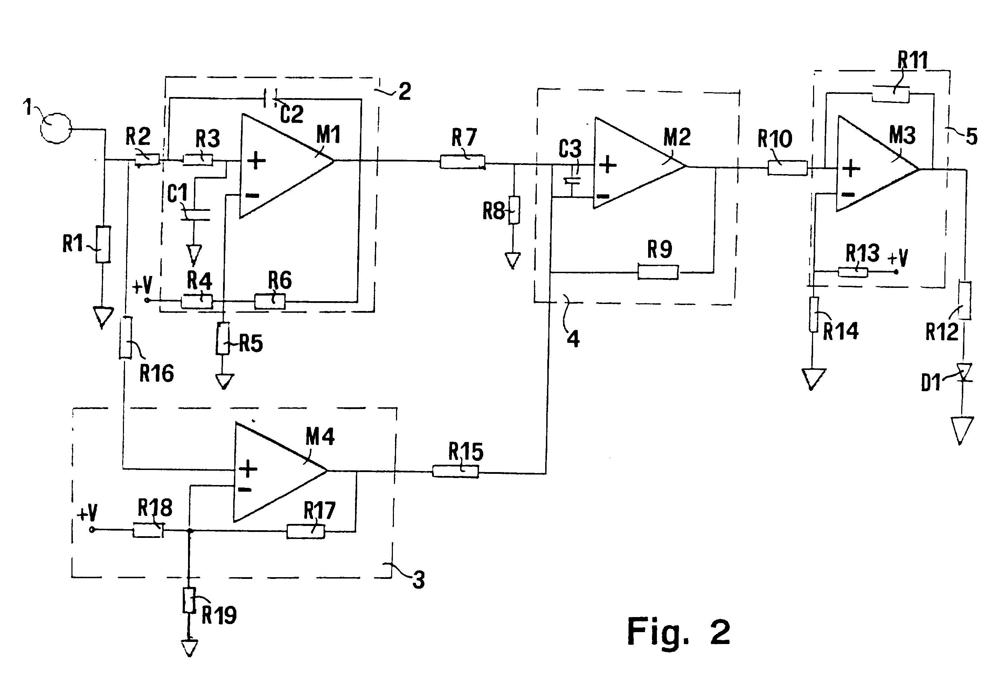

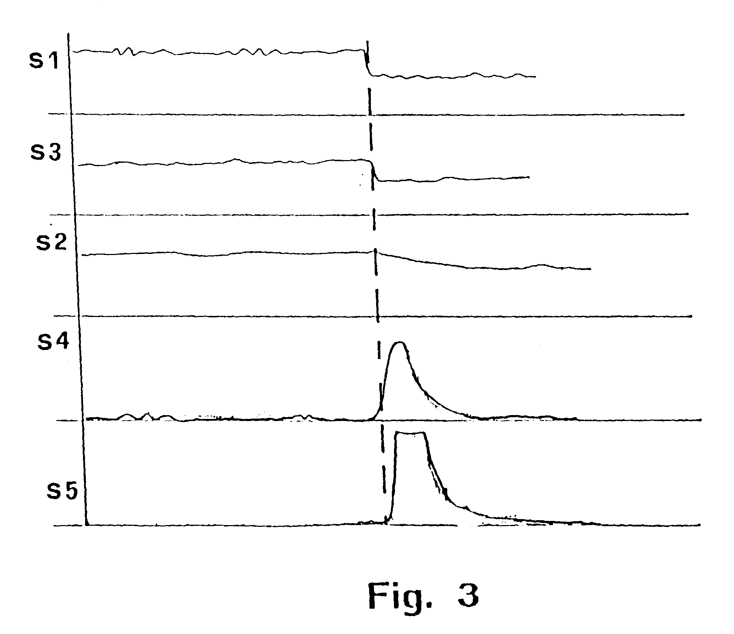

Ces avantages et caractéristiques de l'invention ainsi que d'autres seront plus et mieux compris de chaque homme du métier à la lumière de la description qui va suivre et à l'aide des dessins annexés donnés à titre d'exemplification pratique de l'invention, mais à ne pas considérer dans le sens limitatif; dessins sur lesquels: la Fig. 1 représente le schéma fonctionnel d'un dispositif, conformément à l'invention; la Fig.2 représente le schéma électrique relatif à une forme préférentielle de réalisation d'un dispositif, conformément à l'invention; la Fig. 3 représente les différents diagrammes des signaux de sortie des éléments opérationnels du dispositif de la Fig. 2. Réduit à sa structure essentielle et en référence aux figures des dessins annexés, le procédé pour la détection de la variation de densité d'un tissu , conformément à l'invention, comporte, dans l'ordre, les phases opérationnelles suivantes:
- déplacement du tissu à traiter par rapport à un capteur optique à réflexion (1) fixe, destiné à détecter la variation de densité du tissu;
- prélèvement du signal de sortie dudit capteur optique (1) et transmission, en parallèle, du signal ainsi prélevé à un circuit de tampon à amplificateurs opérationnels (3) et à des moyens (2) pour calculer la moyenne et retarder ledit signal;
- transmission des signaux de sortie, respectivement du circuit tampon (3) et des moyens (2) pour calculer la moyenne et retarder le signal, à l'entrée d'un amplificateur différentiel (4);
- transmission du signal de sortie dudit amplificateur différentiel (4) à l'entrée d'un comparateur (5) du signal par rapport à un signal connu et prédéterminé;
- transmission du signal de sortie dudit comparateur (5) aux moyens opérationnels pour le traitement successif du tissu, lorsque le signal en sortie dudit amplificateur est d'intensité supérieure à une valeur prédéterminée.

Avantageusement, il est prévu que ledit signal connu et prédéterminé est variable à souhait, en fonction des exigences de l'utilisateur.

Pour ce qui concerne le dispositif pour mettre en oeuvre ledit procédé, conformément à l'invention, celui-ci comprend:
- un capteur optique à réflexion (1) fixe, destiné à détecter une variation de densité d'un tissu, par exemple la ligne de jarretière d'un bas;
- un circuit de tampon à amplificateurs opérationnels (3) avec l'entrée connectée de manière électrique à la sortie du capteur optique (1);
- des moyens (2) pour calculer la moyenne et retarder le signal prélevé à la sortie des moyens optiques (1);
- des moyens (4) d'amplification différentielle des signaux de sortie du circuit tampon (3) et des moyens (2) pour calculer la moyenne et retarder le signal prélevé à la sortie du capteur optique (1);
- des moyens (5) pour comparer le signal en sortie des moyens d'amplification différentielle (4) avec un signal connu et prédéterminé;
- des moyens, non représentés sur le figures par simplification, pour utiliser le signal de sortie des moyens de comparaison (5) lorsque le signal de sortie des moyens d'amplification différentielle (4) est d'intensité supérieure à une valeur prédéterminée, par exemple pour arrêter le mouvement relatif du bas par rapport au capteur.

De manière plus détaillée et en référence à la Fig. 2 des dessins annexés, une résistance (R1) est connectée en dérivation sur le point de connexion de la sortie du capteur optique (1) avec l'entrée du circuit tampon (3).
Les moyens (2) pour calculer la moyenne et retarder le signal de sortie du capteur optique (1) sont connectés de manière électrique à la sortie du capteur optique (1) au moyen d'une résistance (R2) en série et comprennent un filtre passe-bas (M1) pour permettre uniquement le passage des signaux de fréquences connues et prédéterminées, avec un condensateur (C2) connecté en parallèle entre la sortie de la résistance (R2) et la sortie desdits moyens (2), avec une résistance (R3) en aval du point de connexion du condensateur (C2) avec la sortie de la résistance (R2), cette résistance (R3) étant en série avec la résistance (R2), avec un condensateur (C1) connecté en dérivation sur la sortie de la résistance (R3), avec une résistance (R4) connectée à une de ses bornes à une source de tension à 24 Volt et à l'autre borne à l'entrée négative des moyens (2), avec une résistance (R6) connectée en série entre la sortie de la résistance (R4) et la sortie du condensateur (C2) et avec une résistance (R5) connectée en dérivation sur le point de connexion de la résistance (R4) avec la résistance (R6).

Le circuit tampon à amplificateurs opérationnels (3) est connecté à la sortie du capteur optique (1) au moyen d'une résistance (R16) en série et il est muni d'une résistance (R18) alimentée à une de ses bornes avec une tension de 24 Volt et connectée en série avec une résistance (R17) connectée en dérivation entre l'entrée et la sortie du circuit tampon (3) et avec une résistance (R19) connectée en dérivation sur le point de connexion de la résistance (R17) avec la résistance (R18).

Les moyens (4) d'amplification différentielle comprennent un amplificateur différentiel (M1) avec la borne d'entrée positive connectée, au moyen d'une résistance (R7), avec la sortie des moyens (2) pour calculer la moyenne et retarder le signal provenant de la cellule photoélectrique (1) et connectée en outre, à travers une résistance (R15), avec la sortie du circuit tampon (3).

Avantageusement, l'entrée positive et l'entrée négative de l'amplificateur différentiel (M2) sont connectées au moyen d'un condensateur (C3).

Une résistance (R9) est connectée à une de ses bornes avec la sortie de la résistance (R15) et à l'autre borne avec la sortie de l'amplificateur (M1), alors qu'une résistance (R8) est connectée en dérivation sur le point de connexion de la résistance (R7) avec la borne d'entrée positive de l'amplificateur (M2).

Les moyens (5) de comparaison du signal provenant de l'amplificateur différentiel (M2) avec un signal connu et prédéterminé comprennent un comparateur (M3) à seuil variable pour le réglage du seuil d'intervention. Le comparateur (M3) est avantageusement connecté en cascade avec la sortie de l'amplificateur différentiel (M2) au moyen d'une résistance (R10) et il est muni d'une résistance (R11) avec les deux bornes connectées respectivement avec l'entrée positive et avec la sortie de ce même comparateur (M3).

L'entrée négative du comparateur (M3) est connectée avec une borne d'une résistance (R14): au point de connexion de l'entrée négative du comparateur (M3) avec la résistance (R14) étant connectée une des bornes d'une résistance (R13), laquelle est connectée, à son autre borne, au pôle positif d'une source de tension à 24 Volt. A la sortie du comparateur (7) est connectée en série une résistance (R12) avec une diode (D1) en cascade.

A titre d'exemplification, en référence à une forme préférentielle de réalisation de l'invention, ci-dessous sont reportées les mesures relatives aux éléments de circuit de la Fig. 2:
R1 = 1 kohm
R2, R3, R4, R5, R6, R7 = 100 kohm
R8, R9 = 470 kohm
R10 = 100 kohm
R11 = 1 Mohm
R12 = 2,2 kohm
R13 = 100 kohm
R14 = 47 kohm
R15, R16, R17, R18, R19 = 100 kohm
C1 = 1 uF
C2 = 47 kpF
C3 = 10 kpF
M1, M2, M3, M4 type: LM324
D1 type: 1N4148

Le fonctionnement est le suivant. Le tissu est entraîné par rapport au capteur optique (1), lequel produit un signal électrique qui est transmis en parallèle au filtre passe-bas (2) et au circuit tampon (3). Le signal qui alimente le filtre passe-bas est ainsi transformé en moyenne alors que celui transmis au circuit tampon (3) est retransmis inaltéré. Les signaux en sortie du filtre passe-bas (2) et du circuit tampon (3) sont transmis à l'amplificateur différentiel (4) qui permet d'amplifier la différence entre le signal de moyenne et le signal, instantané, en sortie du circuit tampon (3). Le signal en sortie de l'amplificateur différentiel est ainsi transmis au comparateur (5) qui permet de confronter le signal en entrée avec un signal de référence connu et prédéterminé. Si une inégalité se produit entre le signal de sortie de l'amplificateur et ledit signal de référence connu, le comparateur pourvoit à l'activation des moyens correspondants pour le traitement du tissu: cette inégalité se produisant lorsque le capteur optique (1) détecte la présence d'une différence de densité ou de coloration du tissu et, par conséquent, générant un signal électrique d'intensité et de fréquence correspondant à l'intensité et à la fréquence du signal pour lequel le comparateur (5) est réglé.

## Revendications

1. Procédé pour la détection de la variation de densité d'un tissu , comportant le déplacement du tissu à traiter par rapport à un capteur optique à réflexion, caractérisé en ce qu'il comporte, dans l'ordre, les phases opérationnelles suivantes:
- prélèvement du signal de sortie dudit capteur optique (1) et transmission du signal, en parallèle, à un circuit de tampon à amplificateurs opérationnels (3) et à des moyens (2) pour calculer la moyenne et retarder ledit signal;
- transmission des signaux de sortie, respectivement du circuit tampon (3) et des moyens (2) pour calculer la moyenne et retarder le signal provenant du capteur optique (1), à l'entrée d'un amplificateur différentiel (M2);
- transmission du signal de sortie dudit amplificateur différentiel (M2) à l'entrée d'un comparateur (M3) du signal par rapport à un signal connu et prédéterminé;
- transmission du signal de sortie dudit comparateur (M3) à des moyens opérationnels correspondants pour le traitement successif du tissu, lorsque le signal en sortie dudit amplificateur (M2) est d'intensité supérieure à une valeur prédéterminée.

2. Procédé selon la revendication 1, caractérisé en ce que ledit signal connu et prédéterminé est variable en fonction du type de tissu et des exigences liées à son traitement.

3. Dispositif pour la détection de discontinuités sur un tissu pour la mise en oeuvre du procédé selon la revendication 1 et avec un capteur optique à réflexion (1) pour la détection de la variation de densité du tissu, caractérisé en ce qu'il comprend:
- un circuit de tampon à amplificateurs opérationnels (3) avec l'entrée connectée de manière électrique à la sortie du capteur optique (1);
- des moyens (2) pour calculer la moyenne et retarder le signal prélevé à la sortie du capteur optique (1);
- des moyens (4) d'amplification différentielle des signaux de sortie, respectivement, du circuit tampon (3) et des moyens (2) pour calculer la moyenne et retarder le signal prélevé à la sortie du capteur optique (1);
- des moyens (5) pour comparer le signal de sortie des moyens d'amplification différentielle (4) avec un signal connu et prédéterminé.

4. Dispositif selon la revendication 3, caractérisé en ce que lesdits moyens (2) pour calculer la moyenne et retarder le signal prélevé à la sortie des moyens optiques (1) comprennent un filtre passe-bas (M1).

5. Dispositif selon la revendication 3, caractérisé en ce que lesdits moyens d'amplification différentielle (4) comprennent un amplificateur différentiel (M2).

6. Dispositif selon la revendication 3, caractérisé en ce que lesdits moyens de comparaison (5) comprennent un comparateur à seuil variable (M3).

## Patentansprüche

1. Verfahren zum Detektieren der Veränderung der Dichte eines Gewebes, bei dem das zu behandelnde Gewebe bezüglich eines optischen Reflexionssensors verschoben wird, dadurch **gekennzeichnet**, daß es folgende, der Reihe nach auszuführende Schritte umfaßt:
- Übernahme des Ausgangssignals des optischen Sensors (1) und parallele Übertragung des Signals an eine Pufferschaltung mit Operationsverstärkern (3) und Einrichtungen (2) zur Mittelwertberechnung und zum Verzögern des Signals,
- Übertragung der Ausgangssignale der Pufferschaltung (3) und der Einrichtung (2) zur Mittelwertbildung und zum Verzögern des vom optischen Sensor (1) kommenden Signals an den Eingang eines Differenzverstärkers (M2),
- Übertragen des Ausgangssignals des Differenzverstärkers (M2) an den Eingang eines Komparators (M3) zum Vergleich des Signals mit einem bekannten und vorbestimmten Signal, und
- Übertragung des Ausgangssignals des Komparators (M3) an die entsprechenden Arbeitseinrichtungen zum nachfolgenden Bearbeiten des Gewebes wenn das Ausgangssignal des Verstärkers (M2) eine einen vorgegebenen Wert übersteigende Intensität aufweist.

2. Verfahren nach Anspruch 1, dadurch **gekennzeichnet,** daß das bekannte und vorbestimmte Signal in Abhängigkeit von der Gewebeart und den an seine Bearbeitung zu stellenden Anforderungen veränderbar ist.

3. Vorrichtung zum Detektieren von Diskontinuitäten an einem Gewebe zur Durchführung des Verfahrens nach Anspruch 1 und mit einem optischen Reflexionssensor (1) zum Detektieren der Veränderung der Dichte des Gewebes, dadurch **gekennzeichnet**, daß sie folgende Bestandteile umfaßt:
- eine Pufferschaltung mit Operationsverstärkern (3), deren Eingang in elektrisch leitender Weise mit dem Ausgang des optischen Sensors (1) verbunden ist,
- Einrichtungen (2) zur Mittelwertberechnung und zum Verzögern des am Ausgang des optischen Sensors (1) abgegriffenen Signals,
- Einrichtungen (4) zum differentiellen Verstärken der Ausgangssignale der Pufferschaltung (3) bzw. der Einrichtungen (2) zur Mittelwertberechnung und zum Verzögern des am Ausgang des optischen Sensors (1) abgegriffenen Signals, und
- Einrichtungen (5) zum Vergleichen des Ausgangssignals der Einrichtungen (4) zum differentiellen Verstärken mit einem bekannten und vorbestimmten Signal.

4. Vorrichtung nach Anspruch 3, dadurch **gekennzeichnet**, daß die Einrichtungen (2) zur Mittelwertberechnung und zum Verzögern des am Ausgang der optischen Einrichtung (1) abgegriffenen Signals ein Tiefpaßfilter (M1) umfassen.

5. Vorrichtung nach Anspruch 3, dadurch **gekennzeichnet**, daß die Einrichtungen (4) zum differentiellen Verstärken einen Differenzverstärker (M2) umfassen.

6. Vorrichtung nach Anspruch 3, dadurch **gekennzeichnet**, daß die Vergleichseinrichtungen (5) einen Komparator (M3) mit veränderlichem Schwellenwert umfassen.

## Claims

1. Method for detecting density differences in a fabric, comprising the step of moving the fabric to be treated relative to an optical reflecting sensor, characterized by the fact that further includes the following operating steps:
- picking up the signal on output from said optical sensor (1) and sending, in parallel, the signal to an operational amplifier buffer (3) and to means (2) for averaging and delaying said signal;
- sending the signals on output respectively from the buffer (3) and from the means for averaging and delaying the signal on output from the sensor (1), to a differential amplifier (M2);
- sending the output signal from the differential amplifier to (M2) to the input of a comparator (M3) which compares this signal with a known and predetermined signal;
- sending the signal from the comparator (M3) to operative means for the subsequent treatment of the fabric when the output signal from the amplifier (M2) has an intensity exceeding a predetermined value.

2. Method according to claim 1, characterized by the fact that said known and predetermined signal is variable according to the type of the fabric and to the treatment requirements.

3. Device for detecting density differences in a fabric according to the method of claim 1 and comprising an optical reflecting sensor (1) for sensing the variation of the fabric density, characterized by the fact that it is provided with:
- an operational amplifier buffer (3) with the input electrically connected to the output of the optical sensor (1);
- means (2) for averaging and delaying the output signal of the optical sensor (1);
- means (4) for the differential amplification of the signal outputting from the buffer (3) and from the means (2) for averaging and delaying the output signal of the optical sensor (1) ;
- means (5) for comparing the output signal from the differential amplification means (4) with a known and predetermined signal.

4. Device according to claim 3, characterized by the fact that said means (2) for averaging and delaying the output signal of the optical sensor (1) include a low-pass filter (M1).

5. Device according to claim 3, characterized by the fact that said differential amplification means (4) include a differential amplifier (M2).

6. Device according to claim 3, characterized by the fact that said comparing means (5) include a variable thresold comparator (M3).
